# EUROPEAN PATENT APPLICATION

(11) **EP 4 162 951 A1**
(43) Date of publication of application: **12.04.2023**
(21) Application number: 21816711.2
(22) Date of filing: 04.06.2021
(51) Int. Cl.: A61K 39/395, C12N 9/24, C07K 16/32

(54) **PHARMACEUTICAL COMPOSITION OF ENZYMES AND VIRUSES AND APPLICATION THEREOF**

(30) Priority: 05.06.2020 CN 202010507997
(71) Applicant: Shanghai Bao Pharmaceuticals Co., Ltd., Shanghai 201908 (CN)
(72) Inventor: LIU, Yanjun, Shanghai 201908 (CN); WANG, Zheng, Shanghai 201908 (CN); XU, Yunxia, Shanghai 201908 (CN)
(74) Representative: Pharma Patents International AG
(86) International application number: PCT/CN2021/098263
(87) International publication number: WO 2021/244628

(57) **Abstract**

Disclosed in the present invention is a pharmaceutical composition, comprising: 1) a reagent for reducing bonding between an Fc receptor and an endogenous serum antibody, wherein the reagent comprises an immunoglobulin degrading enzyme or endo-glycosidase; and 2) a viral vector drug, wherein the viral vector drug is selected from an oncolytic virus and a viral vaccine. The pharmaceutical composition allows individual administration of the viral vector drug and the reagent. Further disclosed in the present invention are an application of the pharmaceutical composition in the preparation of a drug for treating or preventing disasters, and a method for applying the pharmaceutical composition to a subject to treat or prevent cancers or infections.

## Description

### Technical Field

The present invention relates to the field of biomedicine, in particular to a pharmaceutical combination of an enzyme and a virus and use thereof.

### Background Art

Currently, viruses are commonly used therapeutic vectors, such as oncolytic viruses, viral vaccines and gene therapy viruses. Commonly used viruses include HSV-1, adenoviruses, etc. A major problem with the application of these viruses to treatment is that most people have been infected with such viruses and have immunogenicity, and neutralizing antibodies thereof are widely present in the population. The positive rate of pre-existing neutralizing antibodies against HSV-1 is considerable. It is one of the main limitations of viral vector therapy that these neutralizing antibodies can block the effects of the viruses. In addition, after one administration of viral vector therapy, the titer of neutralizing antibodies will increase, and the viral vector therapy can no longer be administered within one year, which is another major obstacle to limiting viral vector therapy. At present, immunosuppressants are mainly used to solve this problem, but such treatment methods are not effective.

Oncolytic viruses are a type of viruses that preferentially infect and kill tumor cells. In the initial stage, some tumor cells are specifically infected and destroyed by oncolytic viruses. Subsequently, the oncolytic viruses replicate and proliferate in the tumor cells, and release new infectious virus particles to infect and destroy other tumor cells. The oncolytic viruses exert their oncolytic effects by directly dissolving the tumor cells or stimulating the host to produce an anti-tumor immune response.

Oncolytic viruses are administered by both intratumoral injection and intravenous injection. At present, intratumoral injection has become the mainstream mode of administration, because it is not easy to cause drug resistance, it can also stimulate the immune response to tumor microenvironment, and it is not easy to cause cytokine storm. However, for advanced tumors that have metastasized throughout the body, the effects of intratumoral injection are limited, and intratumoral injection can not achieve the effects of eliminating metastasized tumors. Therefore, the research and development on intravenous injection of oncolytic viruses is more and more than that on intratumoral injection of oncolytic viruses. However, at present, the problem with neutralizing antibodies and safety concerns brought by systematic injection are the main obstacles to intravenous injection of oncolytic viruses. (Russell, S., Peng, K. & Bell, J. Oncolytic virotherapy. Nat Biotechnol 30, 658-670, 2012. https://doi.org/10.1038/nbt.2287). Therefore, there is an urgent need for therapeutics that can reduce or eliminate the obstacles to intravenous injection of oncolytic viruses.

Pre-existing neutralizing antibodies in the human body are also one of the treatment obstacles encountered by viral vector vaccines. In her latest clinical trial article on Ad5-nCoV, a novel coronavirus vaccine with adenovirus as the vector, academician Chen Wei pointed out that pre-existing Ad5 immunity can slow down the rapid immune response to SARS-CoV-2 and reduce the peak level of the response. Furthermore, the high level of Ad5 immunity may also negatively impact the persistence of the vaccine-induced immune response. (Zhu Feng-Cai, Li Yu-Hua, Guan Xu-Hua et al. Safety, tolerability, and immunogenicity of a recombinant adenovirus type-5 vectored COVID-19 vaccine: a dose-escalation, open-label, non-randomised, first-in-human trial. [J]. Lancet, 2020). Therefore, there is an urgent need for enzymes that can safely and effectively degrade IgG for enzymatically digesting IgG in the serum of subjects treated with the Ad5-nCoV vaccine.

WO 2020102740A2 discloses a method of gene therapy, which is a treatment method for enzymatically digesting IgG or sugar chains contained in IgG in the serum of subjects treated with AAV using IdeS or EndoS. However, in the field of oncolytic viruses and viral vaccines, there is an urgent lack of enzymes that effectively degrade sugar chains contained in the antibody Fc portion or IgG.

### Summary of the Invention

In view of the shortcomings of the prior art, the purpose of the present invention is to provide an agent for reducing the interference of IgG in the blood to virus therapy, including a pharmaceutical combination of an immunoglobulin-degrading enzyme or an endoglycosidase and a viral vector drug and use thereof, wherein the viral vector drug comprises an oncolytic virus and a viral vaccine. For oncolytic viruses, the main obstacles to intravenous administration of oncolytic viruses are removed through the pre-removal of neutralizing antibodies related to viral vector drugs in the human body, and/or the removal of neutralizing antibodies produced after administration of viral vectors. For viral vaccines, the effects of neutralizing antibodies are eliminated through the pre-removal of neutralizing antibodies against viral vector drugs.

Specifically, the present invention relates to the following items:
1. A pharmaceutical combination, comprising: 1) an agent for reducing the binding of an Fc receptor to an endogenous serum antibody, wherein the agent comprises an immunoglobulin-degrading enzyme or an endoglycosidase; and 2) a viral vector drug, wherein the viral vector drug is selected from an oncolytic virus and a viral vaccine; and wherein the pharmaceutical combination allows separate administration of the viral vector drug and the agent.
2. The pharmaceutical combination according to item 1, wherein the immunoglobulin-degrading enzyme is an IgG-degrading enzyme, and the IgG-degrading enzyme is selected from a *Streptococcus pyogenes-*derived IgG cysteine protease or a variant or fragment thereof or a human-derived MMP protease or a variant or fragment thereof, wherein the variant or fragment retains the activity of enzymatically digesting IgG; preferably, the IgG-degrading enzyme is selected from IdeS, MAC2, IdeZ, IdeZ2, IdeE, IdeE2, IdeP and MMP.
3. The pharmaceutical combination according to item 1, wherein the endoglycosidase is an IgG endoglycosidase, and the IgG endoglycosidase is selected from an IgG endoglycosidase or a variant or fragment thereof derived from *Streptococcus* spp., such as *Streptococcus pyogenes, Streptococcus equi* or *Streptococcus zooepidemicus, Corynebacterium pseudotuberculosis, Enterococcus faecalis,* or *Elizabethkingia meningosepticum,* wherein the variant or fragment retains the activity of the IgG endoglycosidase; preferably, the IgG endoglycosidase is EndoS, CP40, EndoE or EndoF2.
4. The pharmaceutical combination according to item 2, wherein the IgG-degrading enzyme comprises an amino acid sequence as set forth in any one of SEQ ID NOs: 1-41, or is a protein consisting of the amino acid sequence.
5. The pharmaceutical combination according to item 3, wherein the IgG endoglycosidase comprises an amino acid sequence as set forth in any one of SEQ ID NOs: 42-45, or is a protein consisting of the amino acid sequence.
6. The pharmaceutical combination according to item 1, wherein in the viral vector drug, the virus used in the viral vector drug is selected from an ssDNA virus, a dsDNA virus, an ssRNA virus or a dsRNA virus; and/or the virus used in the viral vector drug is selected from a wild-type virus strain or naturally attenuated strain, a genetically engineered and selective attenuated strain, a gene-loaded virus strain and a gene transcription-targeting virus strain.
7. The pharmaceutical combination according to item 6, wherein the wild-type virus strain or naturally attenuated strain is selected from Newcastle disease virus, reovirus, mumps virus, West Nile virus, adenovirus, vaccinia virus, etc.
8. The pharmaceutical combination according to item 6, wherein the genetically engineered and selective attenuated strain enables the virus to selectively replicate in a tumor by manually deleting a key gene, and the genetically engineered and selective attenuated strain is, for example, ONYX-015 or G207.
9. The pharmaceutical combination according to item 6, wherein the gene-loaded virus strain is loaded with an exogenous gene, such as one of granulocyte-macrophage colony stimulating factor (GM-CSF), and the gene-loaded virus strain is, for example, JX-594 or T-VEC.
10. The pharmaceutical combination according to item 6, wherein the gene transcription-targeting virus strain enables to control the replication of the oncolytic virus in a tumor cell by inserting a tissue- or tumor-specific promoter upstream of an essential gene of the virus, and the gene transcription-targeting virus strain is, for example, G92A.
11. The pharmaceutical combination according to item 6, wherein the ssDNA virus is selected from parvovirus, such as H-1PV virus.
12. The pharmaceutical combination according to item 6, wherein the dsDNA virus is selected from herpes simplex virus, adenovirus and poxvirus; preferably, the adenovirus is selected from Enadenotucirev, DNX-2401, C-REV, NG-348, ProsAtak, CG0070, ADV-TK, EDS01, KH901, H101, H103, VCN-01 and Telomelysin (OBP-301); the herpes simplex virus is preferably herpes simplex virus type I (HSV-1), and is selected from R3616, T-VEC, HF10, G207, NV1020 and OrienX010; and the poxvirus is selected from Pexa-Vec (a vaccinia virus), JX-594 (a vaccinia virus), GL-ONC1 and Myxoma.
13. The pharmaceutical combination according to item 6, wherein the ssRNA virus is selected from picornavirus, alphavirus, retrovirus, paramyxovirus and rhabdovirus; preferably, the picornavirus is selected from CAVATAK, PVS-RIPO, CVA21 (an enterovirus) and RIGVIR, the alphavirus is selected from M1, Sindbis AR339 and Semliki Forest virus, the retrovirus is selected from Toca511, the paramyxovirus is selected from MV-NIS and PV701 (a Newcastle disease virus), and the rhabdovirus is selected from VSV-IFNβ, MG1-MAGEA3 and VS V-GP.
14. The pharmaceutical combination according to item 6, wherein the dsRNA virus is selected from reovirus; preferably, the reovirus is selected from Pelareorep, Reolysin, vaccinia virus, mumps virus and human immunodeficiency virus (HIV).
15. The pharmaceutical combination according to item 6, wherein the ssRNA virus is selected from reovirus, coxsackievirus, poliovirus, porcine Seneca Valley virus, measles virus, Newcastle disease virus, vesicular stomatitis virus (VSV) and influenza virus.
16. The pharmaceutical combination according to item 1, wherein the oncolytic virus expresses an exogenous gene selected from those of a Bispecific T cell engager (BiTE), GM-CSF, interleukin-2 (IL-2), interleukin-12 (IL-12), an interferon (IFN), a tumor necrosis factor (TNF), soluble CD80 and CCL3.
17. The pharmaceutical combination according to any one of items 1-16, wherein the pharmaceutical combination further comprises a targeted drug or a chemotherapeutic drug or an immune checkpoint blocker, wherein the targeted drug is selected from an epigenetic drug, such as a histone deacetylase inhibitor, an inhibitor targeting the PI3K/Akt/mTOR signaling pathway, such as Tricibine, and a tyrosine kinase inhibitor, such as sunitinib; the chemotherapeutic drug is selected from an immunosuppressant, such as cyclophosphamide, gemcitabine, temozolomide, mitoxantrone and bortezomib, and a proteasome inhibitor; and the immune checkpoint blocker is selected from an anti-CTLA-4 antibody, an anti-PD-1 antibody and an anti-TIM-3 antibody.
18. Use of the pharmaceutical combination according to any one of items 1-17 in the preparation of a medicament for treating or preventing a disease, wherein the agent is administered to a subject by intravenous infusion or subcutaneous injection, and/or the amount of the agent administered is from 0.01 mg/kg body weight to 2 mg/kg body weight, from 0.04 mg/kg body weight to 2 mg/kg body weight, from 0.12 mg/kg body weight to 2 mg/kg body weight, from 0.24 mg/kg body weight to 2 mg/kg body weight or from 1 mg/kg body weight to 2 mg/kg body weight; preferably, the disease is a cancer, a viral infection, a bacterial infection or a fungal infection.
19. The use according to item 18, wherein the administration interval of the agent and the viral vector drug is at least 30 minutes, at least 1 hour, at least 2 hours, at least 3 hours, at least 4 hours, at least 4 hours, at least 5 hours or at least 6 hours, and at most 35 days, at most 28 days, at most 21 days, at most 18 days, at most 14 days, at most 13 days, at most 12 days, at most 11 days, at most 10 days, at most 9 days, at most 8 days, at most 7 days, at most 6 days, at most 5 days, at most 4 days, at most 3 days, at most 2 days, at most 24 hours, at most 18 hours, at most 12 hours, at most 10 hours, at most 8 hours, at most 7 hours or at most 6 hours.
20. The use according to item 18, wherein the administration interval of the agent and the viral vector drug is from 30 minutes to 1 hour, from 30 minutes to 2 hours, from 30 minutes to 3 hours, from 30 minutes to 4 hours, from 30 minutes to 5 hours, from 30 minutes to 6 hours, from 1 to 2 hours, from 1 to 3 hours, from 1 to 4 hours, from 1 to 5 hours, from 1 to 6 hours, from 2 to 3 hours, from 2 to 4 hours, from 2 to 5 hours, from 2 to 6 hours, from 3 to 4 hours, from 3 to 5 hours, from 3 to 6 hours, from 4 to 5 hours, from 4 to 6 hours or from 5 to 6 hours.
21. The use according to item 18, wherein the agent is administered before administration of the viral vector drug, or the agent is administered after administration of the viral vector drug.
22. The use according to item 19, wherein when the agent is administered before administration of the viral vector drug, a viral vector-binding antibody in the blood of the subject is quantitatively detected before administration of the agent, after administration of the agent and before administration of the viral vector drug, and after administration of the viral vector drug, to confirm an antibody-mediated effector function.
23. The use according to item 20, wherein when the agent is administered after administration of the viral vector drug, a viral vector-binding antibody in the blood of the subject is quantitatively detected before administration of the viral vector drug, after administration of the viral vector drug and before administration of the agent, and after administration of the agent, to confirm an antibody-mediated effector function.
24. A method for treating or preventing a cancer or an infection, comprising administering the pharmaceutical combination according to any one of items 1-17 to a subject, wherein the method results in a reduction of a viral vector drugbinding antibody by 20-50%, 50-75%, 75-90%, 90-95% or 95% or more; and the infection is preferably a viral infection, a bacterial infection or a fungal infection.
25. The method according to item 24, wherein the viral vector drug is an oncolytic virus; preferably, the cancer is selected from prostate cancer, breast cancer, bladder cancer, colon cancer, rectal cancer, pancreatic cancer, ovarian cancer, lung cancer, cervical cancer, endometrial cancer, renal (renal cell) carcinoma, esophageal cancer, thyroid cancer, lymphoma, skin cancer, melanoma and leukemia.
26. The method according to item 24, wherein the viral vector drug is a viral vaccine; preferably, the viral vaccine is used for targeting or treating prostate cancer, breast cancer, bladder cancer, colon cancer, rectal cancer, pancreatic cancer, ovarian cancer, lung cancer, cervical cancer, endometrial cancer, renal (renal cell) carcinoma, esophageal cancer, thyroid cancer, lymphoma, skin cancer, melanoma, leukemia or a disease caused by a coronavirus or a novel coronavirus.
27. The method according to any one of items 24-26, wherein the components of the pharmaceutical combination are administered separately.
28. The method according to any one of items 24-26, wherein the components of the pharmaceutical combination are administered simultaneously.

### Brief Description of the Drawings

Figure 1 shows the electrophoretogram of IdeS and IdeE expression, 1 - molecular weight marker, 2 - pre-induction, 3 - IdeS, and 4 - IdeE.
Figures 2 and 3 show the electrophoretogram of enzymatic digestion of IgG1 by IdeS and IdeE *in vitro,* respectively. The arrows on the right side of the figures indicate different cleavage products from IgG. Arrow 1: intact IgG1; Arrow 2: scIgG 1 (single-cleaved IgG1 - produced by cleavage of the first IgG heavy chain); and Arrow 3: F(ab')2 fragment (produced by cleavage of the second IgG heavy chain).
Figures 4 and 5 show the electrophoretogram of enzymatic digestion of IVIg by IdeS and IdeE *in vitro,* respectively.
Figure 6 shows the virus infection of different groups of mice.
Figure 7 shows the detection of neutralizing antibodies in the sera of mice.
Figure 8 shows the tumor volume growth curve of different groups of mice.

### Detailed Description of Embodiments

The immunoglobulin-degrading enzyme provided by the present invention can enzymatically digest immunoglobulins in the blood effectively, and there is no antibody against the enzyme in the human body, and therefore it is safe to use. In order to solve the above technical problems, the following are the technical solutions of the present invention:

### I. Pharmaceutical combinations

In a first aspect, there is provided a pharmaceutical combination, comprising: 1) an agent for reducing the binding of an Fc receptor to an endogenous serum antibody, wherein the agent comprises an immunoglobulin-degrading enzyme or an endoglycosidase; and 2) a viral vector drug, wherein the viral vector drug is selected from an oncolytic virus and a viral vaccine; and wherein the pharmaceutical combination allows separate administration of the antibody and the agent. Preferably, the pharmaceutical combination comprises a therapeutically effective amount of the immunoglobulin-degrading enzyme and the viral vector drug. Preferably, the pharmaceutical combination is a pharmaceutical composition and further comprises a pharmaceutically acceptable carrier or diluent.

### 1.1. Immunoglobulin-degrading enzymes or endoglycosidases

Preferably, in the pharmaceutical combination as described above, the immunoglobulin-degrading enzyme is an IgG-degrading enzyme, and the IgG-degrading enzyme is selected from a *Streptococcus pyogenes-*derived IgG cysteine protease or a variant or fragment thereof or a human-derived MMP protease or a variant or fragment thereof, wherein the variant or fragment retains the activity of enzymatically digesting IgG; preferably, the IgG-degrading enzyme is selected from IdeS, MAC2, IdeZ, IdeZ2, IdeE, IdeE2, IdeP and MMP.

Preferably, in the pharmaceutical combination as described above, the endoglycosidase is an IgG endoglycosidase, and the IgG endoglycosidase is selected from an IgG endoglycosidase or a variant or fragment thereof derived from *Streptococcus* spp., such as *Streptococcus pyogenes, Streptococcus equi* or *Streptococcus zooepidemicus, Corynebacterium pseudotuberculosis, Enterococcus faecalis,* or *Elizabethkingia meningosepticum,* wherein the variant or fragment retains the activity of the IgG endoglycosidase; preferably, the IgG endoglycosidase is EndoS, CP40, EndoE or EndoF2.

Preferably, the IgG-degrading enzyme comprises the amino acid sequence of any one of SEQ ID NOs: 1-41 or a variant thereof having the same function, or is a protein or a fragment thereof consisting of the amino acid sequence. In one embodiment, the IgG-degrading enzyme comprises the amino acid sequence of any one of SEQ ID NOs: 1-41, or can further comprise: 1) the amino acid sequence of any one of SEQ ID NOs: 1-41; 2) a variant thereof, which has at least 50% identity to the amino acid sequence of any one of SEQ ID NOs: 1-41 and which has the activity of the IgG-degrading enzyme; or a fragment of 1) and 2), which has the activity of the IgG-degrading enzyme. SEQ ID NOs: 7-41 are IgG-degrading enzyme mutants disclosed in CN 107532156A and CN 107532158A.

Preferably, the IgG endoglycosidase comprises the amino acid sequence of any one of SEQ ID NOs: 42-45 or a variant thereof having the same function, or is a protein consisting of the amino acid sequence. In one embodiment, the IgG endoglycosidase comprises: 1) the amino acid sequence of any one of SEQ ID NOs: 42-45; 2) a variant thereof, which has at least 50% identity to the amino acid sequence of any one of SEQ ID NOs: 42-45 and which has the activity of the IgG endoglycosidase; or a fragment of 1) and 2), which has the activity of the IgG endoglycosidase.

In a fourth aspect of the present invention, there is provided a composition, especially a pharmaceutical composition or a pharmaceutical combination, comprising the mutant or protein as described above and a therapeutic agent, wherein the therapeutic agent can be any therapeutic agent that has produced an anti-drug antibody or is prone to produce an anti-drug antibody in the body after administration, including but not limited to an antibody drug, a fusion protein, a small molecule drug, a nucleic acid drug, an antibody-drug conjugate or a viral vector drug; and further comprising a pharmaceutically acceptable carrier or excipient.

### 1.2. Viral vector drugs

Preferably, in the viral vector drug of the pharmaceutical combination as described above, the virus used in the viral vector drug is selected from an ssDNA virus, a dsDNA virus, an ssRNA virus or a dsRNA virus; and/or the virus used in the viral vector drug is selected from a wild-type virus strain or naturally attenuated strain, a genetically engineered and selective attenuated strain, a gene-loaded virus strain and a gene transcription-targeting virus strain.

Preferably, the wild-type virus strain or naturally attenuated strain is selected from Newcastle disease virus, reovirus, mumps virus, West Nile virus, adenovirus, vaccinia virus, etc.

Preferably, the genetically engineered and selective attenuated strain enables the virus to selectively replicate in a tumor by manually deleting a key gene, such as thymidine kinase (TK)-knockout genetically modified human herpes simplex virus I (HSV-1), and the genetically engineered and selective attenuated strain is, for example, ONYX-015 or G207. In ONYX-015, a segment of 827 bp in the E1b region is deleted, and a point mutation is made at the gene for protein E1B55K, so that its expression is terminated prematurely, and the E1B55K protein can not be expressed. In G207, the γ34.5 gene is deleted, which is the determinant of HSV-1 neurotoxicity.

Preferably, the gene-loaded virus strain is loaded with an exogenous gene, such as one of granulocyte-macrophage colony stimulating factor (GM-CSF), and the gene-loaded virus strain is, for example, JX-594 or T-VEC.

Preferably, the gene transcription-targeting virus strain enables to control the replication of the oncolytic virus in a tumor cell by inserting a tissue- or tumor-specific promoter upstream of an essential gene of the virus, and the gene transcription-targeting virus strain is, for example, G92A.

Preferably, in the pharmaceutical combination as described above, the ssDNA virus is selected from parvovirus, preferably H-1PV virus.

Preferably, the dsDNA virus is selected from herpes simplex virus, adenovirus and poxvirus; more preferably, the herpes simplex virus is preferably herpes simplex virus type I (HSV-1), such as R3616, T-VEC, HF10, G207, NV1020 and OrienX010; the poxvirus is selected from Pexa-Vec (a vaccinia virus), JX-594 (a vaccinia virus), GL-ONC1 and Myxoma; and the adenovirus is selected from Enadenotucirev, DNX-2401, C-REV, NG-348, ProsAtak, CG0070, ADV-TK, EDS01, KH901, H101, H103, VCN-01 and Telomelysin (OBP-301).

Preferably, the ssRNA virus is selected from picornavirus, alphavirus, retrovirus, paramyxovirus and rhabdovirus; preferably, the picornavirus is selected from CAVATAK, PVS-RIPO, CVA21 (an enterovirus) and RIGVIR, the alphavirus is selected from M1, Sindbis AR339 and Semliki Forest virus, the retrovirus is selected from Toca511, the paramyxovirus is selected from MV-NIS and PV701 (a Newcastle disease virus), and the rhabdovirus is selected from VSV-IFNβ, MG1-MAGEA3 and VSV-GP.

Preferably, the dsRNA virus is selected from reovirus; preferably, the reovirus is selected from Pelareorep, Reolysin, vaccinia virus, mumps virus and human immunodeficiency virus (HIV). Preferably, the RNA virus is selected from reovirus, coxsackievirus, poliovirus, porcine Seneca Valley virus, measles virus, Newcastle disease virus, vesicular stomatitis virus and influenza virus.

Preferably, in the pharmaceutical combination as described above, the oncolytic virus expresses an exogenous gene, preferably those of a Bispecific T cell engager (BiTE), an scFv fragment, a cytokine and a chemokine. The BiTE can bind to a molecule that activates T cells such as CD3, and can also bind to an antigen target on the surface of a cancer cell. The scFv targets an immune checkpoint, including CTLA-4, PD-1, TIM-3, LAG3, Siglec15, 4-1BB, GITR, OX40, CD40L, CD28, TIGIT and VISTA. The cytokine and chemokine are, for example, GM-CSF, interleukin-2 (IL-2), interleukin-12 (IL-12), an interferon (IFN), a tumor necrosis factor (TNF), soluble CD80 and CCL3.

### 1.3. Drug targets

Preferably, in the pharmaceutical combination as described above, the drug target can be a cell surface protein, including but not limited: AFP, αv integrin, α4β7 integrin, BCMA, CD2, CD3, CD19, CD20, CD22, CD25, CD30, CD32, CD33, CD36, CD40, CD46, CD52, CD56, CD64, CD70, CD74, CD79, CD80, CD86, CD105, CD121, CD123, CD133, CD138, CD174, CD205, CD227, CD326, CD340, CEA, c-Met, Cripto, CA1X, Claudin18.2, ED-B, EGFR, EpCAM, EphA2, EphB2, FAP, FOLR1, GD2, Globo H, GPC3, GPNMB, HER-1, HER-2, HER-3, MAGE-A3, mesothelin, MUC16, GPNMB, PSMA, TMEFF2, TAG-72, 5T4, ROR-1, Sca-1, SP, VEGF or WT1.

The antibody drug target can be a cytokine, including but not limited to: interleukins IL-1 to IL-13, tumor necrosis factors α and β, interferons α, β and γ, tumor growth factor β (TGF-β), colony stimulating factor (CSF) or granulocytemonocyte colony stimulating factor (GM-CSF). See Human Cytokines: Handbook for Basic & Clinical Research (Aggrawal et al. (eds), Blackwell Scientific, Boston, MA 1991).

The antibody drug target can be a hormone, an enzyme, and an intracellular and intercellular messenger, such as adenylate cyclase, guanylate cyclase or phospholipase C.

The antibody drug target can be an immune checkpoint, including: CTLA-4, PD-1, PD-L1, TIM-3, LAG3, Siglec15, 4-1BB, GITR, OX40, CD40L, CD28, TIGIT and VISTA.

### 1.4. Other drugs

Preferably, in any of the pharmaceutical combinations as described above, the pharmaceutical combination further comprises a targeted drug or a chemotherapeutic drug or an immune checkpoint blocker, wherein the targeted drug is selected from an epigenetic drug, such as a histone deacetylase inhibitor, an inhibitor targeting the PI3K/Akt/mTOR signaling pathway, such as Tricibine, and a tyrosine kinase inhibitor, such as sunitinib; the chemotherapeutic drug is selected from an immunosuppressant, such as cyclophosphamide, thalidomide and pomalidomide, a proteasome inhibitor, such as bortezomib, a cytotoxic drug, such as gemcitabine and temozolomide, and a cell cycle non-specific drug, such as mitoxantrone; and the immune checkpoint blocker is selected from an anti-CTLA-4 antibody, an anti-PD-1 antibody, an anti-TIM-3 antibody, an anti-LAG3 antibody, an anti-Siglec15 antibody, an anti-4-1BB antibody, an anti-GITR antibody, an anti-OX40 antibody, an anti-CD40L antibody, an anti-CD28 antibody, an anti-TIGIT antibody and an anti-VISTA antibody.

### II. Uses of pharmaceutical combinations

The present invention also provides use of any of the pharmaceutical combinations in the preparation of a medicament for treating or preventing a disease. Preferably, the disease is a cancer or an infectious disease. The infectious disease comprises a viral infection, a bacterial infection or a fungal infection.

### 2.1. Cancers

The cancer is selected from the group consisting of: acute lymphoblastic leukemia, acute myeloid leukemia, adrenocortical carcinoma, AIDS-related cancers, AIDS-related lymphomas, anal carcinoma, appendiceal cancer, astrocytoma, childhood cerebellar or cerebral cancer, basal cell carcinoma, extrahepatic cholangiocarcinoma, bladder cancer, bone cancer, osteosarcoma/malignant fibrous histiocytoma, brainstem glioma, brain cancer, brain tumor - cerebellar astrocytoma, brain tumor - cerebral astrocytoma/malignant glioma, brain tumor - ependymoma, brain tumor - medulloblastoma, brain tumor - supratentorial primitive neuroectodermal tumor, brain tumor - visual pathway and hypothalamic glioma, breast cancer, bronchial adenoma/carcinoid, Burkitt lymphoma, carcinoid tumor, gastrointestinal carcinoid tumor, cancer of unknown primary, central nervous system lymphoma, cerebellar astrocytoma, cerebral astrocytoma/malignant glioma, cervical cancer, chronic lymphocytic leukemia, chronic myeloid leukemia, chronic myeloproliferative disorders, colon cancer, cutaneous T-cell lymphoma, connective tissue proliferative small round cell tumor, endometrial cancer, ependymoma, esophageal cancer, Ewing's sarcoma in the Ewing's tumor family, extracranial germ cell tumor, children extragonadal germ cell tumor, extrahepatic biliary tract cancer, eye cancer - intraocular melanoma, eye cancer - retinoblastoma, gallbladder carcinoma, gastric cancer, gastrointestinal carcinoid tumor, gastrointestinal stromal tumor (GIST), extracranial, extragonadal or ovarian germ cell tumor, gestational trophoblastic tumor, brainstem glioma, childhood cerebral neuroastrocytoma, children visual pathway and hypothalamic glioma, gastric carcinoid, hairy cell leukemia, head and neck cancer, heart cancer, hepatocellular (hepatic) carcinoma, Hodgkin's lymphoma, hypopharyngeal cancer, hypothalamus and visual pathway glioma, intraocular melanoma, islet cell carcinoma (endocrine pancreas), Kaposi sarcoma, renal carcinoma (renal cell carcinoma), laryngeal carcinoma, leukemia, acute lymphoblastic leukemia (also known as acute lymphocytic leukemia), acute myeloid leukemia (also known as acute myelogenous leukemia), chronic lymphocytic leukemia (also known as chronic lymphatic leukemia), chronic myelogenous leukemia (also known as chronic myeloid leukemia), hairy cell leukemia, lip and oral cancer, liposarcoma, hepatic carcinoma (primary), non-small cell lung cancer, small cell lung cancer, lymphoma, AIDS-related lymphomas, Burkitt lymphoma, cutaneous T-cell lymphoma, Hodgkin's lymphoma, non-Hodgkin's lymphoma (old classification: all other lymphomas except for Hodgkin's lymphoma), primary central nervous system lymphoma, macroglobulinemia, malignant fibrous histiocytoma of bone/osteosarcoma, medulloblastoma, melanoma, intraocular (ocular) melanoma, Merkel cell carcinoma, mesothelioma, adult malignant mesothelioma, metastatic squamous neck cancer with occult primary, oral cancer, multiple endocrine neoplasia syndrome, multiple myeloma/plasma cell tumor, granuloma fungoides, myelodysplastic syndrome, myelodysplasia/myeloproliferative diseases, chronic myelogenous leukemia, adult acute myeloid leukemia, childhood acute myeloid leukemia, multiple myeloma (bone marrow cancer), myeloproliferative disorders, nasal cavity and paranasal sinus cancer, nasopharyngeal carcinoma, neuroblastoma, non-Hodgkin's lymphoma, non-small cell lung cancer, oral cancer, oropharyngeal carcinoma, osteosarcoma/malignant fibrous histiocytoma of bone, ovarian cancer, ovarian epithelial cancer (superficial epithelial-mesenchymal tumor), ovarian germ cell tumor, low malignant potential ovarian tumor, pancreatic cancer, pancreatic islet cell carcinoma, paranasal sinus and nasal cavity cancer, parathyroid carcinoma, carcinoma of penis, pharyngeal carcinoma, pheochromocytoma, pineal gland astrocytoma, pineal gland germ cell tumor, pinealoblastoma and supratentorial primitive neuroectodermal tumor, pituitary tumor, plasmacytoma/multiple myeloma, pleuropulmonary blastoma, primary central nervous system lymphoma, prostate cancer, rectal cancer, renal cell carcinoma of the renal pelvis and ureter(renal carcinoma), transitional cell carcinoma, retinoblastoma, rhabdomyosarcoma, salivary gland carcinoma, sarcomas in the Ewing's tumor family, Kaposi sarcoma, soft tissue sarcoma, uterine sarcoma, Sézary syndrome, skin cancer (non-melanoma), skin cancer (melanoma), Merkel cell skin cancer, small cell lung cancer, small intestinal carcinoma, soft tissue sarcoma, squamous cell carcinoma, squamous neck cancer with occult primary, metastatic gastric cancer, supratentorial primitive neuroectodermal tumor, cutaneous T-cell lymphoma (see granuloma fungoides and Sézary syndrome), carcinoma of testis, throat cancer, thymoma, thymoma and thymic carcinoma, thyroid cancer, thyroid cancer, transitional cell carcinoma of the renal pelvis and ureter, trophoblastic tumor of the ureter and renal pelvis, transitional cell carcinoma of the urethra, endometrial cancer of the uterus, uterine sarcoma, vaginal cancer, visual pathway and hypothalamic glioma, vulvar carcinoma, macroglobulinemia and nephroblastoma (renal carcinoma).

### 2.2. Cancers or infectious diseases

The cancer or infectious disease can be an animal disease or a human disease, for example:

| Recombinant viral vectors | Targeted cancers and infectious diseases | |
|---|---|---|
| | Animals | Humans |
| Adenovirus | Avian influenza virus, *Mycobacterium tuberculosis* and foot-and-mouth disease virus | *Plasmodium falciparum, Mycobacterium tuberculosis,* influenza virus, HIV-1, hepatitis C virus and coronavirus |
| *Shigella* bacteriophage | None | *Mycobacterium tuberculosis* |
| Canarypox attenuated virus | Equine influenza virus, West Nile virus, rabies virus, feline leukemia virus and canine distemper virus | HIV-1 and cancers |
| Fowlpox virus | Avian influenza virus, fowlpox virus and Newcastle disease virus | Cancers |
| Newcastle disease virus | Avian influenza virus and Newcastle disease virus | None |
| Herpesvirus of turkeys | Infectious bursal disease virus and Marek's disease virus | None |
| Attenuated yellow fever virus strain 17D | West Nile virus | West Nile virus, dengue virus and Japanese encephalitis virus |
| Lentivirus | None | Melanoma and HIV-1 |
| Measles virus | None | *Plasmodium falciparum* and human papilloma virus |
| Modified vaccinia virus Ankara | *Mycobacterium bovis* | *Plasmodium falciparum, Mycobacterium tuberculosis,* influenza A virus, colon cancer, renal carcinoma, lung cancer and prostate cancer |
| New York attenuated vaccinia virus | None | *Plasmodium falciparum* and HIV-1 |
| Sendai virus | None | HIV-1 |
| Vaccinia virus | Rabies virus | Cancers |

### III. Treatment methods with pharmaceutical compositions

In the use of the present invention, the agent and the viral vector drug are present as a combined preparation for simultaneous, separate or sequential use.

In some embodiments, the method comprises the steps of: 1) administering the agent to a subject; and subsequently, 2) administering the viral vector drug to the subject. Preferably, the agent is an immunoglobulin-degrading enzyme, and there is an interval for administration of the immunoglobulin-degrading enzyme and the viral vector drug.

In some embodiments, the method comprises the steps of: 1) administering the viral vector drug to the subject; and subsequently, 2) administering the agent to the subject. Preferably, the agent is an immunoglobulin-degrading enzyme, and there is an interval for administration of the immunoglobulin-degrading enzyme and the viral vector drug.

Preferably, the administration amount and interval of the mutant as described above are sufficient to reduce the immunoglobulin level in the subject to 60% of the initial level. More preferably, the administration amount and interval of the agent are sufficient to reduce the immunoglobulin level in the subject to below 50%, 40%, 30%, 20% or 10% of the initial level in the patient. The agent can be administered at a single time point or within a given period of time.

Preferably, the mutant as described above is administered by intravenous infusion, intraperitoneal injection, intramuscular injection, intraarticular injection, intradermal injection or subcutaneous injection, preferably intravenous infusion. Additionally or alternatively, the administration amount of the mutant as described above is from 0.01 mg/kg body weight to 2 mg/kg body weight, from 0.04 mg/kg body weight to 2 mg/kg body weight, from 0.12 mg/kg body weight to 2 mg/kg body weight, from 0.24 mg/kg body weight to 2 mg/kg body weight or from 1 mg/kg body weight to 2 mg/kg body weight.

Preferably, the administration interval of the mutant as described above and the therapeutic agent is at least 30 minutes, at least 1 hour, at least 2 hours, at least 3 hours, at least 4 hours, at least 4 hours, at least 5 hours or at least 6 hours, and at most 35 days, at most 28 days, at most 21 days, at most 18 days, at most 14 days, at most 13 days, at most 12 days, at most 11 days, at most 10 days, at most 9 days, at most 8 days, at most 7 days, at most 6 days, at most 5 days, at most 4 days, at most 3 days, at most 2 days, at most 24 hours, at most 18 hours, at most 12 hours, at most 10 hours, at most 8 hours, at most 7 hours or at most 6 hours.

Preferably, the administration interval of the mutant as described above and the therapeutic agent is from 30 minutes to 1 hour, from 30 minutes to 2 hours, from 30 minutes to 3 hours, from 30 minutes to 4 hours, from 30 minutes to 5 hours, from 30 minutes to 6 hours, from 1 to 2 hours, from 1 to 3 hours, from 1 to 4 hours, from 1 to 5 hours, from 1 to 6 hours, from 2 to 3 hours, from 2 to 4 hours, from 2 to 5 hours, from 2 to 6 hours, from 3 to 4 hours, from 3 to 5 hours, from 3 to 6 hours, from 4 to 5 hours, from 4 to 6 hours or from 5 to 6 hours.

In yet another embodiment, the method comprises the steps of: 1) treating the blood from the subject with the mutant as described above *ex vivo;* 2) returning the blood to the subject; and 3) administering the therapeutic agent to the subject.

In yet another embodiment, the method comprises the steps of: 1) administering the therapeutic agent to the subject; 2) treating the blood from the subject with the mutant as described above *ex vivo;* and 3) returning the blood to the subject.

In a preferred embodiment, the mutant as described above and the therapeutic agent are used for preventing and/or treating a cancer.

In a preferred embodiment, the mutant as described above and the therapeutic agent are used for preventing and/or treating a viral infection.

In a preferred embodiment, the mutant as described above and the therapeutic agent are used for preventing and/or treating a bacterial infection.

In a preferred embodiment, the mutant as described above and the therapeutic agent are used for preventing and/or treating a fungal infection.

The present invention also provides a method for treating or preventing a cancer or an infection, comprising administering the pharmaceutical combination to a subject. The method results in a reduction of a viral vector-binding antibody by 20-50%, 50-75%, 75-90%, 90-95% or 95% or more than 95%. The method results in a reduction of the pathogenic IgG antibody by 20-50%, 50-75%, 75-90%, 90-95% or 95% or more than 95%. Preferably, the medicament is used in a method for treating a cancer, preventing a cancer or preventing an infection. The infection is preferably a viral infection, a bacterial infection or a fungal infection. Preferably, the medicament is used in a method for treating a cancer.

Preferably, the therapeutic agent is a viral vector drug; preferably, the viral vector drug is an oncolytic virus or a viral vaccine.

Preferably, the components of the pharmaceutical combination are administered separately or simultaneously.

### IV. Articles of manufacture

The present invention also provides an article of manufacture, comprising an agent for reducing the IgG level in the blood, including an IgG-degrading enzyme and a viral vector drug, for simultaneous, separate or sequential use in cancer treatment, cancer prevention and/or infection prevention as a combined preparation. Preferably, the combined preparation is used in a method for treating a cancer.

In some embodiments, the cancer is selected from the group consisting of: acute lymphoblastic leukemia, acute myeloid leukemia, adrenocortical carcinoma, AIDS-related cancers, AIDS-related lymphomas, anal carcinoma, appendiceal cancer, astrocytoma, childhood cerebellar or cerebral cancer, basal cell carcinoma, extrahepatic cholangiocarcinoma, bladder cancer, bone cancer, osteosarcoma/malignant fibrous histiocytoma, brainstem glioma, brain cancer, brain tumor - cerebellar astrocytoma, brain tumor - cerebral astrocytoma/malignant glioma, brain tumor - ependymoma, brain tumor - medulloblastoma, brain tumor - supratentorial primitive neuroectodermal tumor, brain tumor - visual pathway and hypothalamic glioma, breast cancer, bronchial adenoma/carcinoid, Burkitt lymphoma, carcinoid tumor, gastrointestinal carcinoid tumor, cancer of unknown primary, central nervous system lymphoma, cerebellar astrocytoma, cerebral astrocytoma/malignant glioma, cervical cancer, chronic lymphocytic leukemia, chronic myeloid leukemia, chronic myeloproliferative disorders, colon cancer, cutaneous T-cell lymphoma, connective tissue proliferative small round cell tumor, endometrial cancer, ependymoma, esophageal cancer, Ewing's sarcoma in the Ewing's tumor family, extracranial germ cell tumor, children extragonadal germ cell tumor, extrahepatic biliary tract cancer, eye cancer - intraocular melanoma, eye cancer - retinoblastoma, gallbladder carcinoma, gastric cancer, gastrointestinal carcinoid tumor, gastrointestinal stromal tumor (GIST), extracranial, extragonadal or ovarian germ cell tumor, gestational trophoblastic tumor, brainstem glioma, childhood cerebral neuroastrocytoma, children visual pathway and hypothalamic glioma, gastric carcinoid, hairy cell leukemia, head and neck cancer, heart cancer, hepatocellular (hepatic) carcinoma, Hodgkin's lymphoma, hypopharyngeal cancer, hypothalamus and visual pathway glioma, intraocular melanoma, islet cell carcinoma (endocrine pancreas), Kaposi sarcoma, renal carcinoma (renal cell carcinoma), laryngeal carcinoma, leukemia, acute lymphoblastic leukemia (also known as acute lymphocytic leukemia), acute myeloid leukemia (also known as acute myelogenous leukemia), chronic lymphocytic leukemia (also known as chronic lymphatic leukemia), chronic myelogenous leukemia (also known as chronic myeloid leukemia), hairy cell leukemia, lip and oral cancer, liposarcoma, hepatic carcinoma (primary), non-small cell lung cancer, small cell lung cancer, lymphoma, AIDS-related lymphomas, Burkitt lymphoma, cutaneous T-cell lymphoma, Hodgkin's lymphoma, non-Hodgkin's lymphoma (old classification: all other lymphomas except for Hodgkin's lymphoma), primary central nervous system lymphoma, macroglobulinemia, malignant fibrous histiocytoma of bone/osteosarcoma, medulloblastoma, melanoma, intraocular (ocular) melanoma, Merkel cell carcinoma, mesothelioma, adult malignant mesothelioma, metastatic squamous neck cancer with occult primary, oral cancer, multiple endocrine neoplasia syndrome, multiple myeloma/plasma cell tumor, granuloma fungoides, myelodysplastic syndrome, myelodysplasia/myeloproliferative diseases, chronic myelogenous leukemia, adult acute myeloid leukemia, childhood acute myeloid leukemia, multiple myeloma (bone marrow cancer), myeloproliferative disorders, nasal cavity and paranasal sinus cancer, nasopharyngeal carcinoma, neuroblastoma, non-Hodgkin's lymphoma, non-small cell lung cancer, oral cancer, oropharyngeal carcinoma, osteosarcoma/malignant fibrous histiocytoma of bone, ovarian cancer, ovarian epithelial cancer (superficial epithelial-mesenchymal tumor), ovarian germ cell tumor, low malignant potential ovarian tumor, pancreatic cancer, pancreatic islet cell carcinoma, paranasal sinus and nasal cavity cancer, parathyroid carcinoma, carcinoma of penis, pharyngeal carcinoma, pheochromocytoma, pineal gland astrocytoma, pineal gland germ cell tumor, pinealoblastoma and supratentorial primitive neuroectodermal tumor, pituitary tumor, plasmacytoma/multiple myeloma, pleuropulmonary blastoma, primary central nervous system lymphoma, prostate cancer, rectal cancer, renal cell carcinoma of the renal pelvis and ureter(renal carcinoma), transitional cell carcinoma, retinoblastoma, rhabdomyosarcoma, salivary gland carcinoma, sarcomas in the Ewing's tumor family, Kaposi sarcoma, soft tissue sarcoma, uterine sarcoma, Sézary syndrome, skin cancer (non-melanoma), skin cancer (melanoma), Merkel cell skin cancer, small cell lung cancer, small intestinal carcinoma, soft tissue sarcoma, squamous cell carcinoma, squamous neck cancer with occult primary, metastatic gastric cancer, supratentorial primitive neuroectodermal tumor, cutaneous T-cell lymphoma (see granuloma fungoides and Sézary syndrome), carcinoma of testis, throat cancer, thymoma, thymoma and thymic carcinoma, thyroid cancer, thyroid cancer, transitional cell carcinoma of the renal pelvis and ureter, trophoblastic tumor of the ureter and renal pelvis, transitional cell carcinoma of the urethra, endometrial cancer of the uterus, uterine sarcoma, vaginal cancer, visual pathway and hypothalamic glioma, vulvar carcinoma, macroglobulinemia and nephroblastoma (renal carcinoma).

The present invention also provides a pharmaceutical composition or therapeutic agent for use in a method of treating a cancer, preventing a cancer and/or preventing an infection, comprising: 1) a therapeutically effective amount of an agent for reducing the IgG level in the blood, including an IgG-degrading enzyme; 2) a therapeutically effective amount of a viral vector drug, preferably an oncolytic virus; and 3) a pharmaceutically acceptable carrier or diluent.

The pharmaceutical composition of the present invention can be used in combination with a targeted drug. The targeted drug is selected from an epigenetic drug, an inhibitor targeting the PI3K/Akt/mTOR signaling pathway and a receptor tyrosine kinase inhibitor.

Histone deacetylase inhibitors (HDACis) are a type of widely studied epigenetic drug, which can not only promote tumor cell differentiation and apoptosis by such means as inhibiting tumor cell proliferation and inducing cell cycle arrest, but also reduce the body's antiviral immune response by inhibiting the interferon signaling pathway. Histone deacetylase inhibitor HDAC6 has been proved to significantly improve the replication level of oncolytic virus HSV-1 in glioma cells, and can cooperate with HSV-1 to kill tumors.

PI3K/Akt signaling pathway is an important signaling pathway to regulate cell proliferation and apoptosis under stress conditions. Akt inhibitor Tricibine can cooperate with oncolytic virus MG18L to induce glioma cell apoptosis, and the combination of the two agents is significantly more effective than each agent alone in the treatment of glioma in mice. Rapamycin is an inhibitor of the mTOR signaling pathway, which can cooperate with adenovirus and HSV-1 to kill nonsusceptible tumor cells.

Protein tyrosine kinase (PTK) inhibitors have multiple effects of inhibiting tumor angiogenesis and resisting tumor cell growth. Sunitinib is a small molecule receptor tyrosine kinase inhibitor, which can improve the replication of oncolytic virus VSV in tumor cells by inhibiting the activity of PTKs in cells, and can also enhance the ability of the oncolytic virus to infect tumors by inhibiting the VEGFR signaling pathway to disrupt tumor angiogenesis, thereby significantly enhancing the therapeutic effects of the oncolytic virus.

The pharmaceutical composition of the present invention can be used in combination with a chemotherapeutic drug. The combined use can kill immunogenic cells, enhance tumor cell antigenicity or susceptibility to immune cells, and inhibit negatively regulatory Treg cells and myeloid-derived suppressor cells (MDSCs). The chemotherapeutic drug is selected from an immunosuppressant, such as cyclophosphamide, thalidomide and pomalidomide, a proteasome inhibitor, such as bortezomib, a cytotoxic drug, such as gemcitabine and temozolomide, and a cell cycle non-specific drug, such as mitoxantrone. Preferably, the combined use is a combination of oncolytic reovirus (RV) and bortezomib (BTZ).

The pharmaceutical composition of the present invention can be used in combination with an immune checkpoint blocker. The immune checkpoint is selected from CTLA-4, PD-1, TIM-3, LAG3, Siglec15, 4-1BB, GITR, OX40, CD40L, CD28, TIGIT and VISTA. The immune checkpoint blocker is selected from an anti-CTLA-4 antibody, an anti-PD-1 antibody, an anti-TIM-3 antibody, an anti-LAG3 antibody, an anti-Siglec15 antibody, an anti-4-1BB antibody, an anti-GITR antibody, an anti-OX40 antibody, an anti-CD40L antibody, an anti-CD28 antibody, an anti-TIGIT antibody and an anti-VISTA antibody.

The present invention also provides a kit or kit of parts for preventing or treating a cancer or an infection, comprising: 1) a therapeutically effective amount of an agent, including the mutant as described above; and 2) a therapeutically effective amount of a therapeutic agent selected from a viral vector drug, an antibody, and a polypeptide drug capable of reducing the IgG level in the blood; preferably, the viral vector drug is an oncolytic virus or a gene therapy virus. The kit can further comprise 3) a targeted drug or a chemotherapeutic drug or an immune checkpoint blocker.

The targeted drug is selected from an epigenetic drug, such as a histone deacetylase inhibitor, an inhibitor targeting the PI3K/Akt/mTOR signaling pathway, such as Tricibine, and a tyrosine kinase inhibitor, such as sunitinib; the chemotherapeutic drug is selected from an immunosuppressant, such as cyclophosphamide, thalidomide and pomalidomide, a proteasome inhibitor, such as bortezomib, a cytotoxic drug, such as gemcitabine and temozolomide, and a cell cycle non-specific drug, such as mitoxantrone; and the immune checkpoint blocker is selected from an anti-CTLA-4 antibody, an anti-PD-1 antibody, an anti-TIM-3 antibody, an anti-LAG3 antibody, an anti-Siglec15 antibody, an anti-4-1BB antibody, an anti-GITR antibody, an anti-OX40 antibody, an anti-CD40L antibody, an anti-CD28 antibody, an anti-TIGIT antibody and an anti-VISTA antibody.

The kit or kit of parts comprises a part A, comprising a therapeutically effective amount of an agent for reducing the immunoglobulin level in the blood, including an IgG-degrading enzyme; and a part B, comprising a therapeutically effective amount of a therapeutic agent selected from a viral vector drug, preferably an oncolytic virus, an antibody, and a polypeptide drug capable of reducing the IgG level in the blood. The kit of parts can further comprise a part C. The part C comprises a targeted drug or a chemotherapeutic drug or an immune checkpoint blocker. The targeted drug is selected from an epigenetic drug, such as a histone deacetylase inhibitor, an inhibitor targeting the PI3K/Akt/mTOR signaling pathway, such as Tricibine, and a tyrosine kinase inhibitor, such as sunitinib; the chemotherapeutic drug is selected from an immunosuppressant, such as cyclophosphamide, thalidomide and pomalidomide, a proteasome inhibitor, such as bortezomib, a cytotoxic drug, such as gemcitabine and temozolomide, and a cell cycle non-specific drug, such as mitoxantrone; and the immune checkpoint blocker is selected from an anti-CTLA-4 antibody, an anti-PD-1 antibody, an anti-TIM-3 antibody, an anti-LAG3 antibody, an anti-Siglec15 antibody, an anti-4-1BB antibody, an anti-GITR antibody, an anti-OX40 antibody, an anti-CD40L antibody, an anti-CD28 antibody, an anti-TIGIT antibody and an anti-VISTA antibody.

The kit can comprise instructions on the administration of the therapeutically effective amount of the agent for reducing the immunoglobulin level in the blood and the therapeutically effective amount of the therapeutic agent (e.g., dose information and administration interval information). The therapeutic agent is selected from a viral vector drug, preferably an oncolytic virus, an antibody, and a polypeptide drug capable of reducing the IgG level in the blood.

In any aspect of the present invention, the agent for reducing the immunoglobulin level in the blood as described above and the therapeutically effective amount of the therapeutic agent can be administered simultaneously, separately or sequentially, for example for use in cancer treatment, cancer prevention and/or treatment, and/or infection prevention and/or treatment. The mutant or protein as described above and the viral vector drug can be provided as separate preparations or as a combined preparation.

As used herein, the term "drug (or agent) for reducing the immunoglobulin level in the blood" preferably refers to a drug or agent that reduces the immunoglobulin level in the blood to 60% or below of the original level. Preferably, the drug or agent reduces the immunoglobulin level in the blood to up to 60% of the original level, up to 50% of the original level, up to 40% of the original level, up to 30% of the original level, up to 20% of the original level, up to 10% of the original level or up to 0% of the original level. More preferably, the drug or agent reduces the immunoglobulin level in the blood to up to 20% of the original level, up to 10% of the original level or up to 0% of the original level.

Well-established expression systems can be used to prepare viral vector drugs. Some examples of methods include the use of mammalian cell expression systems to produce viral particles, such as the use of HEK293 cells to produce adenovirus viral vector drugs (Freedman Joshua D, Duffy Margaret R, Lei-Rossmann Janet et al., An Oncolytic Virus Expressing a T-cell Engager Simultaneously Targets Cancer and Immunosuppressive Stromal Cells. [J].Cancer Res., 2018, 78: 6852-6865).

The pharmaceutical carrier can be liquid, and the pharmaceutical composition can be in the form of a solution. Liquid carriers are used to prepare solutions, suspensions, emulsions, syrups, elixirs and pressurized compositions. The active ingredients can be dissolved or suspended in a pharmaceutically acceptable liquid carrier, such as water, an organic solvent, a mixture of the two, or a pharmaceutically acceptable oil or fat.

The pharmaceutical composition for parenteral administration is sterile, substantially isotonic, and pyrogen-free, and is prepared in accordance with the GMP of the FDA or a similar agency. The viral vector drug can be administered as an injectable dosage form of a solution or suspension thereof, wherein the substance is in physiologically acceptable diluent and pharmaceutical carrier (which can be a sterile liquid, such as water, oil, saline, glycerol or ethanol). In addition, an auxiliary substance such as a wetting agent or an emulsifier, a surfactant and a pH buffering substance can be present in the composition. Other components of the pharmaceutical composition include those of petroleum, animal, plant or synthetic origin, such as peanut oil, soybean oil and mineral oil. In general, diols such as propylene glycol or polyethylene glycol are preferred liquid carriers, especially for injectable solutions. The viral vector drug can be administered in the form of a depot injection or an implanted preparation, which can be formulated to allow sustained release of the active ingredient. Typically, the composition is prepared as an injectable preparation, i.e., a liquid solution or suspension, or can be prepared as a solid form suitable for dissolution or suspension in a liquid carrier prior to injection.

The agent and the oncolytic virus or viral vaccine can be administered by any suitable route. Preferably, both the agent and the oncolytic virus are administered intravenously (i.v.). Preferably, both the agent and the viral vaccine are administered intravenously (i.v.). Preferably, the agent is administered intravenously (i.v.), and the viral vaccine is administered intramuscularly.

Unless otherwise defined, all technical and scientific terms used herein have the same meanings as commonly understood by those of ordinary skill in the art to which the present invention belongs. While any methods, devices and materials similar or equivalent to those described herein can be used in the practice or testing of the present invention, preferred methods, devices and materials are now described.

The terms "polypeptide" and "protein" are used interchangeably herein to mean polymers of amino acid residues. In other words, the description of a polypeptide is equally applicable to the description of a peptide and a protein, and vice versa. The terms apply to naturally occurring amino acid polymers, and amino acid polymers in which one or more amino acid residues are non-naturally encoded amino acids. As used herein, the terms encompass amino acid chains of any length, including full-length proteins (i.e., antigens), in which amino acid residues are linked via covalent peptide bonds.

On the basis of the general knowledge in the art, the above-mentioned preferred conditions can be combined arbitrarily, so that preferred examples of the present invention can be obtained.

The reagents and raw materials used in the present invention are all commercially available.

The beneficial effects of the present invention are at least as follows: By combining immunoglobulin-degrading enzymes or endoglycosidases with oncolytic viruses, on the one hand, there is no interference from neutralizing antibodies against the oncolytic viruses, and on the other hand, cytokine storms and other side effects mediated by the intravenous injection of the oncolytic viruses are also eliminated, which makes the intravenous injection of the oncolytic viruses no longer an obstacle to their administration, making it possible to treat metastatic tumors with oncolytic viruses. By combining immunoglobulin-degrading enzymes or endoglycosidases with viral vaccines, on the one hand, there is no interference from neutralizing antibodies against the vaccine viral vectors, and on the other hand, cytokine storms and other side effects mediated by the intravenous injection of the viral vaccines are also eliminated, which greatly improves the safety of viral vaccines.

### Examples

The present invention will be further described by way of examples below, but the present invention is not limited to the scope of the described examples. The experimental methods in the following examples that do not indicate the specific conditions are selected according to conventional methods and conditions or according to the product's instructions.

### Example 1. Sample preparation

The nucleotide sequences of proteases IdeS and IdeE were obtained by gene synthesis techniques, and recombined into the expression vector pET32a to construct recombinant expression vectors. The correct recombinant expression vectors proved by sequencing were transformed into the expression bacteria BL21 *Escherichia coli,* and the positive expression bacteria containing the gene sequences of proteases IdeS and IdeE were obtained.

The synthesized sequence encoding IdeS is as follows (SEQ ID NO: 46):

GATAGCTTTAGCGCAAACCAGGAGATCCGCTATAGCGAGGTTACCCCGTATCACGTTACCAGCGTTTGGACCAAAG
GCGTTACCCCGCCGGCCAACTTTACCCAGGGCGAAGACGTGTTTCATGCCCCGTATGTTGCCAATCAGGGCTGGTA
CGACATCACCAAAACCTTCAATGGCAAGGACGATCTGCTGTGCGGTGCCGCAACCGCCGGTAACATGCTGCACTGG

TGGTTCGACCAGAATAAAGACCAGATCAAACGCTACCTGGAGGAACACCCGGAAAAACAGAAAATTAATTTCAACG
GCGAACAGATGTTTGATGTGAAAGAAGCTATTGATACCAAGAACCACCAGCTGGACAGCAAGCTGTTCGAATATTT
TAAGGAGAAAGCCTTCCCGTACCTGAGCACCAAACATCTGGGCGTGTTTCCGGACCATGTGATCGACATGTTCATC
AACGGCTATCGCCTGAGCCTGACCAATCATGGTCCGACCCCGGTGAAAGAAGGTAGCAAAGATCCGCGCGGTGGTA
TCTTCGATGCCGTGTTTACACGTGGCGATCAGAGTAAGCTGCTGACCAGCCGCCATGATTTTAAAGAGAAAAATCT
GAAAGAAATCAGCGATCTGATTAAGAAGGAGCTGACCGAGGGCAAAGCCCTGGGCCTGAGCCACACCTACGCCAAT
GTGCGCATCAACCACGTGATCAACCTGTGGGGTGCCGACTTTGATAGCAACGGCAACCTGAAGGCAATTTACGTGA
CCGACAGCGACAGCAATGCCAGTATTGGCATGAAAAAATACTTTGTTGGTGTGAACAGCGCCGGCAAAGTGGCAAT
CAGTGCCAAGGAGATCAAAGAAGATAACATCGGCGCCCAGGTTCTGGGTCTGTTTACCCTGAGCACAGGTCAGGAT
AGCTGGAATCAGACCAATCATCATCACCACCATCACAGTAGTGGT

The synthesized sequence encoding IdeE is as follows (SEQ ID NO: 47):

GATGACTATCAAAGAAACGCGACGGAGGCCTATGCTAAGGAGGTCCCTCATCAAATTACGTCCGTTTGGACTAAGG
GGGTTACCCCACTGACTCCCGAGCAGTTTAGATATAATAACGAAGATGTCATCCATGCCCCATACCTTGCGCACCA
GGGTTGGTACGACATAACAAAGGCTTTTGACGGCAAAGATAACCTTCTGTGTGGTGCGGCAACTGCCGGTAACATG
CTTCATTGGTGGTTCGATCAAAACAAAACAGAAATTGAGGCGTACCTTAGTAAACACCCCGAAAAACAGAAGATCA
TCTTCAACAACCAGGAGCTTTTTGACTTAAAGGCCGCTATTGATACGAAGGATAGTCAGACGAACAGTCAATTATT
TAACTATTTCAGAGACAAAGCGTTCCCAAACCTTAGCGCGCGCCAACTGGGCGTTATGCCTGACCTGGTGTTGGAC
ATGTTCATTAACGGATATTACCTGAATGTATTTAAAACTCAATCGACTGATGTCAACCGCCCTTACCAGGACAAGG
ATAAACGTGGTGGCATATTTGACGCAGTATTTACACGTGGGGACCAAACGACACTTTTAACTGCGCGGCACGACCT
TAAAAACAAGGGTCTGAATGATATCTCAACTATAATTAAGCAGGAACTTACTGAGGGACGCGCATTGGCTTTGTCA
CATACTTACGCAAACGTATCTATTTCCCATGTGATTAACTTGTGGGGGGCCGATTTTAACGCTGAGGGAAATCTGG
AAGCTATCTATGTGACTGACTCCGACGCTAACGCTTCAATCGGGATGAAGAAATACTTCGTGGGAATAAACGCCCA
CCGCCACGTTGCGATAAGCGCCAAAAAGATAGAGGGGGAAAACATAGGCGCACAGGTATTGGGGTTGTTTACACTG
TCATCGGGAAAGGATATTTGGCAAAAGCTGTCGCATCACCATCATCACCAC

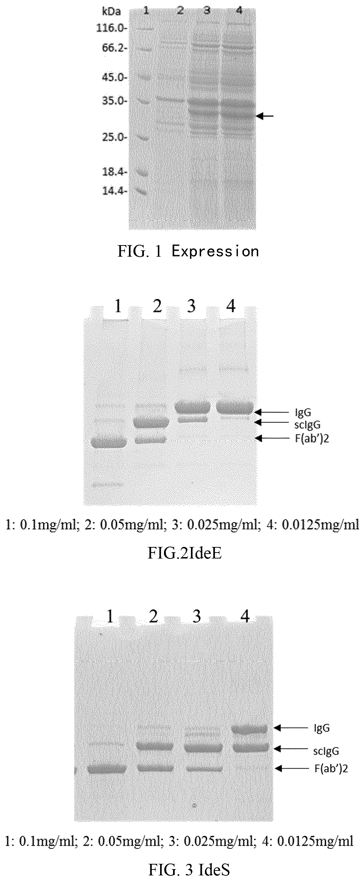

Specifically, six single colonies were picked, and cultured overnight at 37°C. The bacterial solution cultured overnight was added to 5 mL of Amp + LB medium at a ratio of 1:100, and cultured at 37°C and 220 rpm for 2-3 h. 1 mL of the bacterial solution was used as the uninduced control. IPTG was added to the remaining solution by volume until the final concentration was 0.5 mM, and the mixture was cultured at 37°C and 180 rpm for 4 h. The expression products were detected by 12% SDS-PAGE electrophoresis (Figure 1). The expression bacteria were collected, and lysed by conventional methods. Proteases IdeS and IdeE were obtained by purification over a nickel column, and subjected to endotoxin removal and sterilization and filtration for later use.

### Example 2. Assessment of human IgG1-cleaving activity

To test the activity of cleaving human IgG1, trastuzumab was selected as the substrate. IdeS and IdeE were diluted to 0.1 mg/mL, 0.05 mg/mL, 0.025 mg/mL and 0.0125 mg/mL. 1 ul of enzymes of different concentrations was each added to 9 ul of reaction system containing 1 mg/ml of trastuzumab to initiate the cleavage reaction, and the reaction system was placed at 37°C for 30 min. The sample was mixed with an equal volume of 2× SDS loading buffer, and placed in water bath at 75°C for 5 min. The cleavage products were detected by SDS-PAGE. Both IdeS and IdeE significantly cleave human IgG1 (Figures 2-3).

### Example 3. Cleavage of IVIg by IgG-degrading enzymes in vitro

The cleavage activity of IdeE, IdeS or IdeZ on human immunoglobulin IVIg *in vitro* was evaluated by detecting the amount of intact or single-cleaved IVIg after adding protease IdeE, IdeS or IdeZ.

5 ul of diluted proteases IdeE, IdeS and IdeZ (Genovis) was each added to 45 ul of IVIg-containing reaction system at a mass ratio of enzyme to IVIg of 1:200 to initiate the cleavage reaction, in which the experiment of IdeZ was performed on the basis that 10 µg of IVIg was cleaved by 1 Unit of IdeZ. The reaction system was placed at 37°C for 1 h. The sample was mixed with an equal volume of 2× SDS loading buffer, and placed in water bath at 75°C for 5 min. The cleavage products were detected by SDS-PAGE.

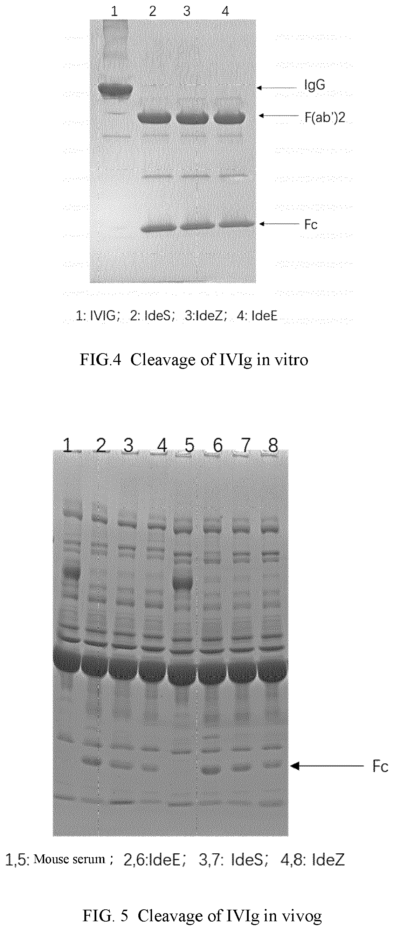

Figure 4 shows the enzymatic digestion of human IVIg by the three enzymes *in vitro.* The electrophoresis results showed that all the three proteases can effectively cleave human IVIg.

### Example 4. Cleavage of IVIg by IgG-degrading enzymes in vivo

Under sterile conditions, eight mice were injected with human IVIg (an intravenous human immunoglobulin) at a dose of 1 g/kg intraperitoneally, two mice in each group (two mice were parallel experiments, numbered No. 1 and No. 2). 24 h after injecting human IVIg, the mice were injected with IgG-degrading enzyme IdeS or IdeE at a dose of 5 mg/kg, or IdeZ at a dose of 1000 units/kg intravenously by group, in which one group was the blank control (normal saline). 24 h after injecting the IgG-degrading enzyme, blood was collected from the two mice in each group, and serum samples were collected, respectively. The serum samples were detected by non-reducing SDS-PAGE. The results showed that IdeS, IdeZ and IdeE significantly cleave IVIg in the mice, and substantially, IVIg can be enzymatically digested completely at 24 h (Figure 5).

Titer detection of neutralizing antibodies against adenovirus: HEK293 cells were plated to a 96-well plate at 5 × 10⁴/well, and the plate was cultured at 37°C and 5% CO₂ for about 7 h. The sera of the mice were diluted at a factor of 10, and then diluted to ten gradients at a 2-fold dilution ratio. The diluents were mixed with an equal volume of Ad5-Luc virus (2 × 10⁴ TU/well), and the mixtures were incubated at room temperature for 1 h, respectively. There were three replicate wells for each diluent, and neither serum nor virus was added to the negative control. After the incubation was completed, the incubated mixture was added to the cell culture plate, and the plate was cultured at 37°C and 5% CO₂ for 24 h. After the culture was completed, Bio-Bright^{™} One-Step firefly luciferase assay kit reagents were added, and the chemiluminescence signal was detected according to the instructions.

Titer detection of neutralizing antibodies against HSV-1 virus: U-2 OS cells were plated to a 96-well plate at 2 × 10⁴/well, and the plate was cultured at 37°C and 5% CO₂ for about 7 h. The sera of the mice were diluted at a factor of 10, and then diluted to ten gradients at a 2-fold dilution ratio. The diluents were mixed with an equal volume of HSV-1 virus (1 × 10⁴ PFU/well), and the mixtures were incubated at room temperature for 1 h, respectively. There were three replicate wells for each diluent, and neither serum nor virus was added to the negative control. The incubated mixture was added to the cell culture plate, and the plate was cultured for 16 h. After the culture was completed, the cells were fixed with 1% paraformaldehyde, then 0.1% Triton X-100 was added, and the plate was incubated at room temperature for 5 min. Subsequently, an anti-HSV1 antibody (10 µg/ml) was added, and the plate was incubated for 1 h. Subsequently, an antimouse IgG-HRP secondary antibody (1:2000 dilution) was used, and the plate was incubated for 30 min. The TMB substrate was added, and the plate was incubated for 15 min. After drying the well plate, the ELISPOT analyzer (CTL) was used to read and analyze the data.

**Table 1. Effect of enzyme on neutralizing antibody titer**

| **No.** | **Enzyme** | **anti-Adv neutralizing antibody** | | **anti-HSV1 neutralizing antibody** | |
|---|---|---|---|---|---|
| | | **Pre-administration** | **Post-administration** | **Pre-administration** | **Post-administration** |
| 1 | Normal saline | 1:320 | 1:320 | 1:1280 | 1:1280 |
| 2 | Normal saline | 1:320 | 1:320 | 1:1280 | 1:1280 |
| 3 | IdeS | 1:320 | <1:10 | 1:1280 | <1:10 |
| 4 | IdeS | 1:320 | <1:10 | 1:1280 | <1:10 |
| 5 | IdeZ | 1:320 | 1:40 | 1:1280 | 1:320 |
| 6 | IdeZ | 1:320 | 1:80 | 1:1280 | 1:320 |
| 7 | IdeE | 1:320 | <1:10 | 1:1280 | <1:10 |
| 8 | IdeE | 1:320 | <1:10 | 1:1280 | <1:10 |

The experiments of titer detection of neutralizing antibodies showed that there are neutralizing antibodies against HSV1 and Adv in IVIg, and IdeS, IdeZ and IdeE can effectively cleave the neutralizing antibodies in the mice.

### Example 5. Effect of IgG-degrading enzymes on infection ability of Adv5 in vivo

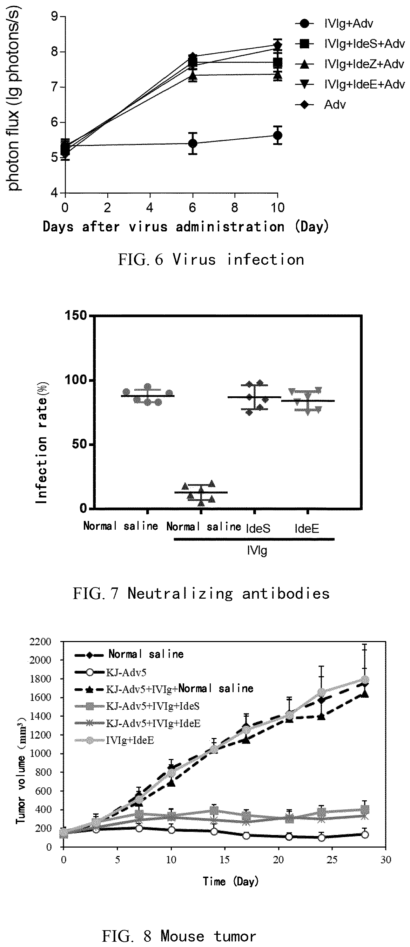

C57BL/6 mice were used to evaluate the effect of IgG-degrading enzymes on the infection ability of Adv5-Luc *in vivo.* The C57BL/6 mice were injected with human IVIg intraperitoneally, six mice in each group. On Day -1, the mice were injected with human IVIg at a dose of 1 g/kg intraperitoneally. 30 min after injecting human IVIg, proteases IdeS and IdeE were administered at a dose of 5 mg/kg intravenously to the mice, and protease IdeZ was injected at a dose of 1000 units/kg intravenously to the mice. On Day 0, Adv5-Luc was administered at 5 × 10¹⁰ vg/mouse, and the fluorescence of the mice was detected on Days 6 and 10. The experimental design is shown in Table 2. The experimental results are shown in Figure 6, indicating that all the three IgG-degrading enzymes can reduce the effect of IVIg on the infection ability of Adv5-Luc virus.

**Table 2. Animal model design of Adv5-Luc**

| Group | | IVIg dose (mg/kg) | Enzyme dose (mg/kg) | Adv5-Luc dose (vg/mouse) |
|---|---|---|---|---|
| 1 | IVIg + Adv | 1000 | / | 5 × 10¹⁰ |
| 2 | IVIg + IdeS + Adv | 1000 | 5 | 5 × 10¹⁰ |
| 3 | IVIg + IdeZ + Adv | 1000 | 1000 units/kg | 5 × 10¹⁰ |
| 4 | IVIg + IdeE + Adv | 1000 | 5 | 5 × 10¹⁰ |
| 5 | Adv | / | / | 5 × 10¹⁰ |

### Example 6. Mouse tumor model

Using the A549 athymic nude mouse tumor model, the effect of the combination of IdeE and KJ-Adv5 adenovirus was compared with that of KJ-Adv5 viral vector drug alone. Female athymic mice aged 6-8 weeks were inoculated with A549 tumor cells (5 × 10⁶) in 0.1 ml of PBS subcutaneously in the area of the right lower extremity for tumorigenesis. When the tumors reached ~150 mm³, the mice were randomly divided into three groups, six mice in each group. On Day -1, blood was collected, and then human IVIg was injected intraperitoneally. 30 min after injecting human IVIg, the proteases were administered intravenously at a dose of 5 mg/kg. On Day 0, blood was collected for the detection of neutralizing antibodies. After collecting blood, KJ-Adv5 was injected at 5 × 10¹⁰ vp/mouse intravenously. Tumor volume was measured twice a week, and tumors were weighed. The mice were sacrificed on Day 28. The experimental design is shown in Table 3.

**Table 3. Doses of IVIg, enzyme and KJ-Adv5 administered in different groups**

| Group | | IVIg dose (mg/kg) | Enzyme dose (mg/kg) | KJ-Adv5 dose (vp/mouse) |
|---|---|---|---|---|
| 1 | Normal saline | / | / | / |
| 2 | KJ-Adv5 | / | / | 5 × 10¹⁰ |
| 3 | KJ-Adv5 + IVIg + normal saline | 1000 | / | 5 × 10¹⁰ |
| 4 | KJ-Adv5 + IVIg + IdeS | 1000 | 5 | 5 × 10¹⁰ |
| 5 | KJ-Adv5 + IVIg + IdeE | 1000 | 5 | 5 × 10¹⁰ |
| 6 | IVIg + IdeE | 1000 | 5 | / |

Detection of neutralizing antibodies: The sera of the mice were diluted at a factor of 10, and then diluted to ten gradients at a 2-fold dilution ratio. The diluents were respectively mixed with Ad5-Luc adenovirus (2 × 10⁴ TU/well), the mixtures were added to a 96-well plate, and the plate was incubated at room temperature for 1 h. There were two replicate wells for each diluent, and neither serum nor virus was added to the negative control. After the incubation was completed, 5 × 10⁴ HEK293 cells were added to each well, and the plate was cultured at 37°C and 5% CO₂ for 24 h. After the culture was completed, Bio-Bright^{™} One-Step firefly luciferase assay kit reagents were added, and the chemiluminescence signal was detected according to the instructions. Figure 7 shows that IVIg contains neutralizing antibodies against the adenovirus, and the neutralizing antibodies can be degraded with IdeS or IdeE.

The experimental results showed that both IdeS and IdeE can eliminate the negative effect of IVIg on the therapeutic effects of intravenous administration of viruses (Figure 8).

The Applicant declares that the detailed methods of the present invention are illustrated by means of the above-mentioned examples, but the present invention is not limited to the detailed methods mentioned above, that is, it does not mean that the present invention must rely on the detailed methods mentioned above to carry out. Those skilled in the art should understand that any improvement of the present invention, the equivalent replacement of each raw material of the articles of manufacture of the present invention, the addition of auxiliary components, the selection of specific methods, etc., fall within the scope of protection and the scope of disclosure of the present invention.

## Claims

1. A pharmaceutical combination, comprising: 1) an agent for reducing the binding of an Fc receptor to an endogenous serum antibody, wherein the agent comprises an immunoglobulin-degrading enzyme or an endoglycosidase; and 2) a viral vector drug, wherein the viral vector drug is selected from an oncolytic virus and a viral vaccine; and wherein the pharmaceutical combination allows separate administration of the viral vector drug and the agent.

2. The pharmaceutical combination according to claim 1, wherein the immunoglobulin-degrading enzyme is an IgG-degrading enzyme, and the IgG-degrading enzyme is selected from a *Streptococcus pyogenes-*derived IgG cysteine protease or a variant or fragment thereof or a human-derived MMP protease or a variant or fragment thereof, wherein the variant or fragment retains the activity of enzymatically digesting IgG; preferably, the IgG-degrading enzyme is selected from IdeS, MAC2, IdeZ, IdeZ2, IdeE, IdeE2, IdeP and MMP.

3. The pharmaceutical combination according to claim 1, wherein the endoglycosidase is an IgG endoglycosidase, and the IgG endoglycosidase is selected from an IgG endoglycosidase or a variant or fragment thereof derived from *Streptococcus* spp., such as *Streptococcus pyogenes, Streptococcus equi* or *Streptococcus zooepidemicus, Corynebacterium pseudotuberculosis, Enterococcus faecalis,* or *Elizabethkingia meningosepticum,* wherein the variant or fragment retains the activity of the IgG endoglycosidase; preferably, the IgG endoglycosidase is EndoS, CP40, EndoE or EndoF2.

4. The pharmaceutical combination according to claim 2, wherein the IgG-degrading enzyme comprises an amino acid sequence as set forth in any one of SEQ ID NOs: 1-41, or is a protein consisting of the amino acid sequence.

5. The pharmaceutical combination according to claim 3, wherein the IgG endoglycosidase comprises an amino acid sequence as set forth in any one of SEQ ID NOs: 42-45, or is a protein consisting of the amino acid sequence.

6. The pharmaceutical combination according to claim 1, wherein in the viral vector drug, the virus used in the viral vector drug is selected from an ssDNA virus, a dsDNA virus, an ssRNA virus or a dsRNA virus; and/or the virus used in the viral vector drug is selected from a wild-type virus strain or naturally attenuated strain, a genetically engineered and selective attenuated strain, a gene-loaded virus strain and a gene transcription-targeting virus strain.

7. The pharmaceutical combination according to claim 6, wherein the wild-type virus strain or naturally attenuated strain is selected from Newcastle disease virus, reovirus, mumps virus, West Nile virus, adenovirus, vaccinia virus, etc.

8. The pharmaceutical combination according to claim 6, wherein the genetically engineered and selective attenuated strain enables the virus to selectively replicate in a tumor by manually deleting a key gene, and the genetically engineered and selective attenuated strain is, for example, ONYX-015 or G207.

9. The pharmaceutical combination according to claim 6, wherein the gene-loaded virus strain is loaded with an exogenous gene, such as one of granulocyte-macrophage colony stimulating factor (GM-CSF), and the gene-loaded virus strain is, for example, JX-594 or T-VEC.

10. The pharmaceutical combination according to claim 6, wherein the gene transcription-targeting virus strain enables to control the replication of the oncolytic virus in a tumor cell by inserting a tissue- or tumor-specific promoter upstream of an essential gene of the virus, and the gene transcription-targeting virus strain is, for example, G92A.

11. The pharmaceutical combination according to claim 6, wherein the ssDNA virus is selected from parvovirus, such as H-1PV virus.

12. The pharmaceutical combination according to claim 6, wherein the dsDNA virus is selected from herpes simplex virus, adenovirus and poxvirus; preferably, the adenovirus is selected from Enadenotucirev, DNX-2401, C-REV, NG-348, ProsAtak, CG0070, ADV-TK, EDS01, KH901, H101, H103, VCN-01 and Telomelysin (OBP-301); the herpes simplex virus is preferably herpes simplex virus type I (HSV-1), and is selected from R3616, T-VEC, HF10, G207, NV1020 and OrienX010; and the poxvirus is selected from Pexa-Vec (a vaccinia virus), JX-594 (a vaccinia virus), GL-ONC1 and Myxoma.

13. The pharmaceutical combination according to claim 6, wherein the ssRNA virus is selected from picornavirus, alphavirus, retrovirus, paramyxovirus and rhabdovirus; preferably, the picornavirus is selected from CAVATAK, PVS-RIPO, CVA21 (an enterovirus) and RIGVIR, the alphavirus is selected from M1, Sindbis AR339 and Semliki Forest virus, the retrovirus is selected from Toca511, the paramyxovirus is selected from MV-NIS and PV701 (a Newcastle disease virus), and the rhabdovirus is selected from VSV-IFNβ, MG1-MAGEA3 and VS V-GP.

14. The pharmaceutical combination according to claim 6, wherein the dsRNA virus is selected from reovirus; preferably, the reovirus is selected from Pelareorep, Reolysin, vaccinia virus, mumps virus and human immunodeficiency virus (HIV).

15. The pharmaceutical combination according to claim 6, wherein the ssRNA virus is selected from reovirus, coxsackievirus, poliovirus, porcine Seneca Valley virus, measles virus, Newcastle disease virus, vesicular stomatitis virus (VSV) and influenza virus.

16. The pharmaceutical combination according to claim 1, wherein the oncolytic virus expresses an exogenous gene selected from those of a Bispecific T cell engager (BiTE), GM-CSF, interleukin-2 (IL-2), interleukin-12 (IL-12), an interferon (IFN), a tumor necrosis factor (TNF), soluble CD80 and CCL3.

17. The pharmaceutical combination according to any one of claims 1-16, wherein the pharmaceutical combination further comprises a targeted drug or a chemotherapeutic drug or an immune checkpoint blocker, wherein the targeted drug is selected from an epigenetic drug, such as a histone deacetylase inhibitor, an inhibitor targeting the PI3K/Akt/mTOR signaling pathway, such as Tricibine, and a tyrosine kinase inhibitor, such as sunitinib; the chemotherapeutic drug is selected from an immunosuppressant, such as cyclophosphamide, gemcitabine, temozolomide, mitoxantrone and bortezomib, and a proteasome inhibitor; and the immune checkpoint blocker is selected from an anti-CTLA-4 antibody, an anti-PD-1 antibody and an anti-TIM-3 antibody.

18. Use of the pharmaceutical combination according to any one of claims 1-17 in the preparation of a medicament for treating or preventing a disease, wherein the agent is administered to a subject by intravenous infusion or subcutaneous injection, and/or the amount of the agent administered is from 0.01 mg/kg body weight to 2 mg/kg body weight, from 0.04 mg/kg body weight to 2 mg/kg body weight, from 0.12 mg/kg body weight to 2 mg/kg body weight, from 0.24 mg/kg body weight to 2 mg/kg body weight or from 1 mg/kg body weight to 2 mg/kg body weight; preferably, the disease is a cancer, a viral infection, a bacterial infection or a fungal infection.

19. The use according to claim 18, wherein the administration interval of the agent and the viral vector drug is at least 30 minutes, at least 1 hour, at least 2 hours, at least 3 hours, at least 4 hours, at least 4 hours, at least 5 hours or at least 6 hours, and at most 35 days, at most 28 days, at most 21 days, at most 18 days, at most 14 days, at most 13 days, at most 12 days, at most 11 days, at most 10 days, at most 9 days, at most 8 days, at most 7 days, at most 6 days, at most 5 days, at most 4 days, at most 3 days, at most 2 days, at most 24 hours, at most 18 hours, at most 12 hours, at most 10 hours, at most 8 hours, at most 7 hours or at most 6 hours.

20. The use according to claim 18, wherein the administration interval of the agent and the viral vector drug is from 30 minutes to 1 hour, from 30 minutes to 2 hours, from 30 minutes to 3 hours, from 30 minutes to 4 hours, from 30 minutes to 5 hours, from 30 minutes to 6 hours, from 1 to 2 hours, from 1 to 3 hours, from 1 to 4 hours, from 1 to 5 hours, from 1 to 6 hours, from 2 to 3 hours, from 2 to 4 hours, from 2 to 5 hours, from 2 to 6 hours, from 3 to 4 hours, from 3 to 5 hours, from 3 to 6 hours, from 4 to 5 hours, from 4 to 6 hours or from 5 to 6 hours.

21. The use according to claim 18, wherein the agent is administered before administration of the viral vector drug, or the agent is administered after administration of the viral vector drug.

22. The use according to claim 19, wherein when the agent is administered before administration of the viral vector drug, a viral vector-binding antibody in the blood of the subject is quantitatively detected before administration of the agent, after administration of the agent and before administration of the viral vector drug, and after administration of the viral vector drug, to confirm an antibody-mediated effector function.

23. The use according to claim 20, wherein when the agent is administered after administration of the viral vector drug, a viral vector-binding antibody in the blood of the subject is quantitatively detected before administration of the viral vector drug, after administration of the viral vector drug and before administration of the agent, and after administration of the agent, to confirm an antibody-mediated effector function.

24. A method for treating or preventing a cancer or an infection, comprising administering the pharmaceutical combination according to any one of claims 1-17 to a subject, wherein the method results in a reduction of a viral vector drugbinding antibody by 20-50%, 50-75%, 75-90%, 90-95% or 95% or more; and the infection is preferably a viral infection, a bacterial infection or a fungal infection.

25. The method according to claim 24, wherein the viral vector drug is an oncolytic virus; preferably, the cancer is selected from prostate cancer, breast cancer, bladder cancer, colon cancer, rectal cancer, pancreatic cancer, ovarian cancer, lung cancer, cervical cancer, endometrial cancer, renal (renal cell) carcinoma, esophageal cancer, thyroid cancer, lymphoma, skin cancer, melanoma and leukemia.

26. The method according to claim 24, wherein the viral vector drug is a viral vaccine; preferably, the viral vaccine is used for targeting or treating prostate cancer, breast cancer, bladder cancer, colon cancer, rectal cancer, pancreatic cancer, ovarian cancer, lung cancer, cervical cancer, endometrial cancer, renal (renal cell) carcinoma, esophageal cancer, thyroid cancer, lymphoma, skin cancer, melanoma, leukemia or a disease caused by a coronavirus or a novel coronavirus.

27. The method according to any one of claims 24-26, wherein the components of the pharmaceutical combination are administered separately.

28. The method according to any one of claims 24-26, wherein the components of the pharmaceutical combination are administered simultaneously.
